# EUROPÄISCHE PATENTSCHRIFT

(11) **EP 2 979 751 B1**
(45) Veröffentlichungstag und Bekanntmachung des Hinweises auf die Patenterteilung: **28.10.2020**
(21) Anmeldenummer: 14179023.8
(22) Anmeldetag: 29.07.2014
(51) Int. Cl.: B01J 2/26, A61K 9/20, C07C 35/12

(54) **VERFAHREN ZUR HERSTELLUNG VON FESTEN KÜHLSTOFFEN**
PROCESS FOR THE MANUFACTURING OF SOLID COOLING AGENTS
PROCÉDÉ POUR LA PRÉPARATION DES AGENTS RÉFRIGÉRÉE SOLIDES

(43) Veröffentlichungstag der Anmeldung: 03.02.2016
(73) Patentinhaber: Symrise AG, 37603 Holzminden (DE)
(72) Erfinder: SIEWERT, Jürgen, 37434 Rollshausen (DE); MICHLER, Michael, 37632 Eimen (DE); NIEKERKEN, Jörk, 37603 Holzminden (DE); LENZ, Oliver, 37170 Uslar (DE); WOLTER, Christian, 31868 Ottenstein (DE)
(74) Vertreter: Fabry, Bernd

(56) Entgegenhaltungen:
- WO-A1-03/101924
- WO-A1-2007/071512
- WO-A1-2008/152009
- WO-A1-2010/095034
- US-A- 3 023 253
- US-A- 3 064 311

## Beschreibung

### GEBIET DER ERFINDUNG

Die Erfindung befindet sich auf dem Gebiet der Kühlstoffe, speziell des Menthols und der Mentholverbindungen, und betrifft ein Verfahren zur Herstellung entsprechender fester Kühlstoffe, insbesondere von Schuppen oder Pastillen, mit verbesserten Lagereigenschaften und verminderter Sublimationsneigung.

### STAND DER TECHNIK

Menthol ist ein natürlich vorkommender Wirkstoff, der beim Kontakt mit Schleimhäuten, speziell der Mundschleimhaut, einen kühlenden Effekt bewirkt. Menthol - und zahlreiche in der Folge entwickelte Mentholverbindungen mit teilweise deutlich gesteigerter Kühlwirkung - finden daher in Pharmazie, Kosmetik und Lebensmittelindustrie breite Anwendung. In natürlichen Quellen, beispielsweise dem Pfefferminzöl, kommt Menthol in Form von vier diastereomeren Enantiomerenpaaren vor, von denen nur die Hauptkomponente, das (-)-Menthol oder L-Menthol die gewünschten geschmacklichen und sonstigen sensorischen Eigenschaften hat, wie bereits **in** J. Am. Chem. Soc, Vol. 39 (8), 1917, S. 1515 bis 1525 beschrieben. So liegen insbesondere die Schmelzpunkte dieser verschiedenen Modifikationen zwischen 33 bis 43 °C wie in Archiv der Pharmazie, 307 (7), 1974, S. 497 bis 503 beschrieben. Der Schmelzpunkt der stabilen α- Modifikation liegt demgemäß bei 42 bis 43 °C.

Wegen dieser Lage der Schmelzpunkte kann L-Menthol sowie die meisten Mentholverbindungen an den Endverbraucher sowohl als in beheizten Behältern flüssig gehaltene Schmelze als auch in Form von Kristallen oder anderen erstarrten Formkörpern, wie Kompaktaten, Pastillen, Schuppen und dergleichen abgegeben werden. Generell weisen alle Feststoffe, die, wie L-Menthol und die dem Menthol strukturell verwandten Stoffe, einen Schmelzpunkt nur knapp oberhalb der Umgebungstemperatur haben, eine starke Neigung auf zu verbacken und zu verklumpen. Die Verarbeitung solchen nicht spezifikationsgerechten Materials ist aber mit erheblichem Zusatzaufwand verbunden. Soll nun reines L-Menthol bzw. Mentholverbindungen, d.h. nicht mit Hilfsmitteln wie z.B. Trennmitteln behandeltes Material als Feststoff verkauft werden, muss entweder durch eine geschlossene Kühlkette oder durch die Art der Formgebung sichergestellt werden, dass das Produkt den Endverbraucher in rieselfähiger Form erreicht.

Kommerziell ist beispielsweise Menthol in Form großer Kristalle verfügbar, die bei einer Länge von 0,5 bis 3 cm eine Dicke von 1 bis 3 mm aufweisen. Sie werden traditionell in kleinen Mengen aus natürlich gewonnenem Pfefferminzöl gezüchtet, in dem das Öl in Trögen oder Wannen über viele Tage in Kühlhäusern zur Kristallisation gebracht wird. Diese Kristalle weisen nur bei geringer Schütthöhe eine gute Rieselfähigkeit auf, verbacken aber zusehends bei erhöhter Last und/oder erhöhter Temperatur. Der technische Aufwand für die Kristallisation, Abtrennung und Reinigung der Kristalle und die geringe Raum- Zeit-Ausbeute eines solchen langwierigen Verfahrens machen es für die großtechnische Anwendung unattraktiv.

Die DE 2530481 betrifft eine Vorrichtung zum Kristallisieren von Substanzen, insbesondere von optisch aktiven Mentholverbindungen, die unter Kristallisationsbedingungen grobe nadel- und balkenförmige Kristalle bilden. Das diskontinuierlich durchzuführende Kristallisationsverfahren wird unter Einsatz eines besonderen Rührwerkes durchgeführt, das ein Zusammenbacken der Kristalle in der Kristallsuspension verhindert. Das Wertprodukt wird abschließend durch eine Zentrifuge isoliert und in einem Trockner getrocknet.

Die beiden Patentschriften US 3,023,253 und US 3,064,311 beschreiben geschupptes L-Menthol sowie ein Verfahren zur Herstellung derartiger Schuppen durch Aufbringen einer Schmelze von L-Menthol auf einer gekühlten Tauchwalze. Falls gewünscht kann die Mentholschmelze zwischen ein Paar gegenläufig rotierender, gekühlter Walzen eingebracht werden. Der auf der Tauchwalze ankristallisierte Mentholfilm wird nachbehandelt, indem er durch Wärmeeintrag getempert und durch Aufbringen zusätzlichen Menthols verstärkt wird. Beide Nachbehandlungen werden simultan mit einer Auftragswalze erreicht. Die so erhaltenen Schuppen weisen zunächst eine gute Rieselfähigkeit auf. Nach längerer Lagerung tritt jedoch eine leichte Verbackung ein, die eine mechanische Auflockerung durch Rütteln des Containers erforderlich macht. Es wird bemerkt, dass diese Verbackung durch eine zwar erwähnte, aber nicht näher charakterisierte poröse Oberfläche und damit einhergehende starke Sublimation des Produkts verursacht wird und dass das so erhaltene Produkt durch Kompaktieren zu Pellets weiterverarbeitet werden kann.

Das Prinzip der weiteren Vergröberung der Primärpartikel durch Kompaktierung wird auch in der DE 10224087 betreffend kompaktiertes Menthol in Form von Menthol- Presslingen sowie ein Verfahren zur Herstellung derselben beschrieben. Hier wird aber nicht auf die Wirkung der Partikelgröße allein abgehoben, sondern darauf, dass die Primärpartikel in einer spezifischen Kristallmodifikation vorliegen müssen. Durch Verpressen von Kristallen, die aus einer Lösungskristallisation oder einer Kühlwalzenverschuppung gewonnen wurden, lassen sich gegen Verbackung stabile Kompaktate erhalten, wenn diese überwiegend aus der thermodynamisch stabilen, erst bei 42,5 °C schmelzenden α-Modifikation bestehen.

Gegenstand der internationalen Patentanmeldung WO 2008 152009 A1 (BASF) ist ein Verfahren zur Herstellung von L-Menthol in fester Form durch Inkontaktbringen einer L-Menthol-Schmelze mit zwei voneinander beabstandeten gekühlten Oberflächen unter Erstarrung der L-Menthol-Schmelze zu L-Menthol in fester Form, wobei man den Kontakt zwischen der erstarrenden L-Menthol-Schmelze und den gekühlten Oberflächen mindestens bis zum Abschluss der Erstarrung aufrecht erhält. Bei diesem Verfahren wird die Kristallisation des Menthols durch eine Kombination aus einem Vorkristallisierer und einem Doppelbandkühler bewirkt. Hierbei wird die Mentholsuspension in den Spalt zwischen zwei gekühlten Flächen gegeben und zum Erstarren bzw. Kristallisieren gebracht.

Den Verfahren des Stands der Technik ist eine Reihe von ernsthaften Nachteilen gemeinsam, nämlich insbesondere eine geringe Lagerstabilität. Die Produkte beginnen unmittelbar nach der Lagerung zu verklumpen, es bilden sich Nadeln infolge Sublimation, die Kristalle brechen infolge unzureichender mechanischer Festigkeit, so dass in Summe letztlich unspezifikationsgemäße Ware resultiert, die zu mannigfaltigen Reklamationen Anlass gibt.

Die Aufgabe der vorliegenden Erfindung hat somit darin bestanden, feste Kühlstoffe, speziell Mentholpastillen, zur Verfügung zu stellen, die bei Lagerung eine deutlich geringere Neigung zum Verklumpen zeigen und insbesondere weniger bruchanfällig sind. Gleichzeitig sollte das Verfahren so geführt werden, dass es bei der Kühlung möglichst wenige Anbackungen und damit nur geringe Produktverluste gibt. Schließlich sollten die so erhaltenen Kompaktate bzw. Pastillen möglichst wenig Restwärme abgeben.

### BESCHREIBUNG DER ERFINDUNG

Gegenstand der Erfindung ist ein Verfahren zur Herstellung von festen Kühlstoffen, bei dem man eine vorgekrätzte Schmelze von Mentholverbindungen durch gleichmäßige Vertropfung auf eine vorgekühlte Fläche aufgibt.

Überraschenderweise wurde gefunden, dass die nach dem erfindungsgemäßen Verfahren erhältlichen festen Kühlstoffe, speziell die Pastillen gegenüber dem Stand der Technik
(a) bei Lagerung und Transport nicht verklumpen und einen deutlich geringeren Anteil an gebrochenen Partikeln aufweisen;
(b) wesentliche geringere Anbackungen auf dem Kühlband hinterlassen;
(c) einen wesentlich geringeren Feinanteil und damit eine höhere Ausbeute besitzen;
(d) zudem gewölbte Oberflächen aufweisen und damit in der Schüttung nur wenige Kontaktpunkte zueinander haben;
(e) ein Oberflächen-zu-Volumenverhältnis aufweisen wodurch die Sublimationsneigung weiter reduziert ist;
(f) eine geringere Neigung zur Brückenbildung aufweisen (geringe Verfestigung bei Lagerung);
(g) eine geringe Restwärmemenge in der Verpackung abgeben.

### Menthol und Mentholverbindungen

Mentholverbindungen die im Sinne der Erfindung eingesetzt werden können sind - neben dem Grundkörper Menthol selber - zu verfestigende Produkte beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate
¹FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird. (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter der Kühlstoffe stellt das Monomenthyl Succinate (FEMA GRAS 3810) dar, das als Stoff bereits 1963 von Brown & Williamson Tobacco Corp. patentiert worden **(**US 3,111,127**)** und als Kühlmittel Gegenstand der Schutzrechte US 5,725,865 und 5,843,466 **(V.Mane Fils)** ist. Sowohl das Succinat als auch das analoge Monomenthyl Glutarate (FEMA GRAS 4006) stellen wichtige Vertreter von Monomenthylestern auf Basis von Di- und Polycarbonsäuren dar:

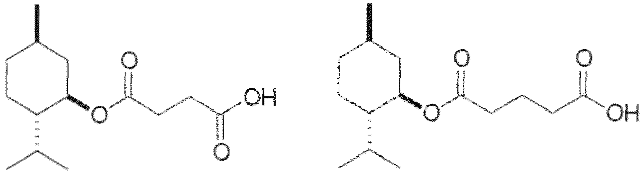

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat® MGC), Menthol Propylenglycol Carbonate (FEMA GRAS 3784 = Frescolat® MPC), Menthol 2-Methyl-1,2-propandiol Carbonate (FEMA GRAS 3849) oder den entsprechenden Zuckerderivaten:

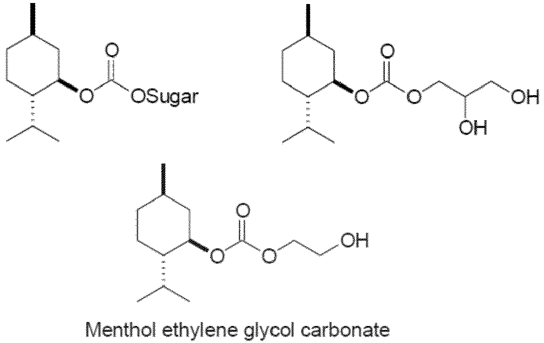

Die Verwendung derartiger Stoffe als Kühlstoff für Zigaretten ist beispielsweise Gegenstand der Druckschrift US 3,419,543 (Mold et al.) aus dem Jahre 1968; die Anwendung als physiologisches Kühlmittel wird in DE 4226043 A1 (H&R) beansprucht.

Im Sinne der Erfindung bevorzugt sind die Mentholverbindungen Menthyl Lactate (FEMA GRAS 3748 = Frescolat® ML) und insbesondere das Menthone Glyceryl Acetal (FEMA GRAS 3807) bzw. Menthone Glyceryl Ketal (FEMA GRAS 3808), das unter der Bezeichnung Frescolat® MGA vermarktet wird.

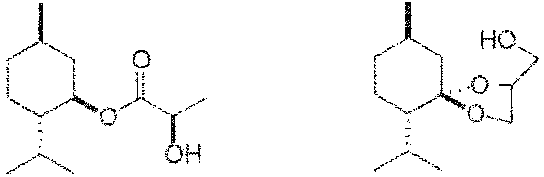

Erstere Struktur wird durch Veresterung von Milchsäure mit Menthol, letztere durch Acetalisierung von Menthon mit Glycerin gewonnen (vgl. DE 2608226 A1**,** H&R). In diese Gruppe von Verbindungen gehört auch das 3-(1-Menthoxy)-1,2,propandiol, das auch als Cooling Agent 10 bekannt ist (FEMA GRAS 3784, vgl. US 6,328,982**,** TIC), sowie das 3-(I-Menthoxy)-2-methyl-1,2,propandiol (FEMA GRAS 3849), das über eine zusätzliche Methylgruppe verfügt.

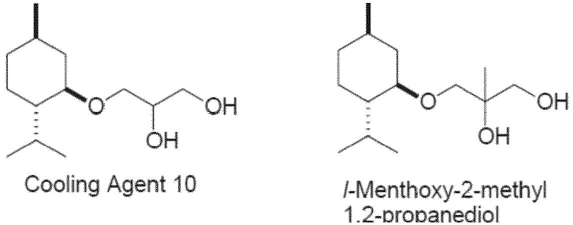

Die Herstellung des 3-(I-Menthoxy)-1,2,propandiol erfolgt beispielsweise ausgehend von Menthol nach dem folgenden Schema (vgl. US 4,459,425, Takagaso):

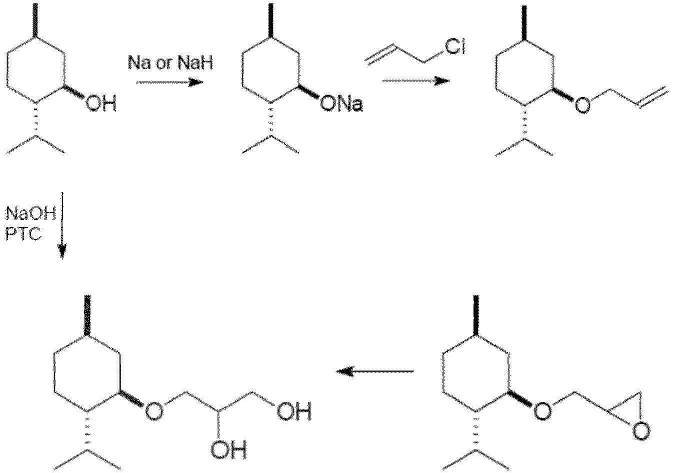

Alternative Routen, bei denen in der ersten Stufe Menthol mit Epichlorhydrin umgesetzt wird, wird in US 6,407,293 und US 6,515,188 (Takagaso) beschrieben. Im Folgenden wird eine Übersicht der bevorzugten Mentholverbindungen gegeben, die sich durch eine CO-Bindung auszeichnen:

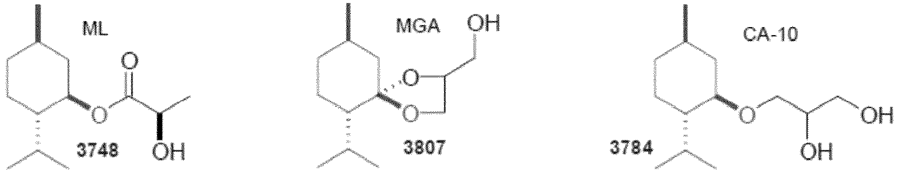

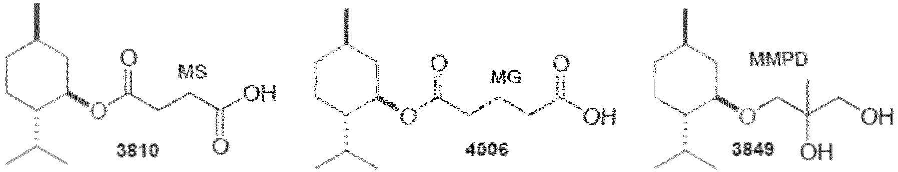

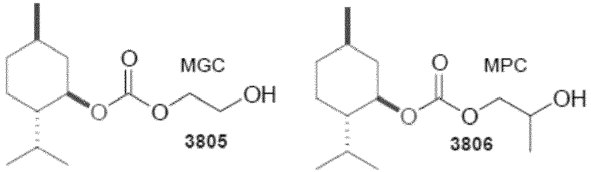

Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonat erwiesen, die die Anmelderin unter den Bezeichnungen Frescolat® MGA, Frescolat® ML, Frescolat® MGC und Frescolat® MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern im Sinne der Erfindung eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die ebenfalls im Sinne der Erfindung bevorzugten Spezies WS-3, WS-4, WS-5, WS-12, WS-14 und WS-30. Die beiden nachfolgenden Schaubilder zeigen die Synthesewege auf:

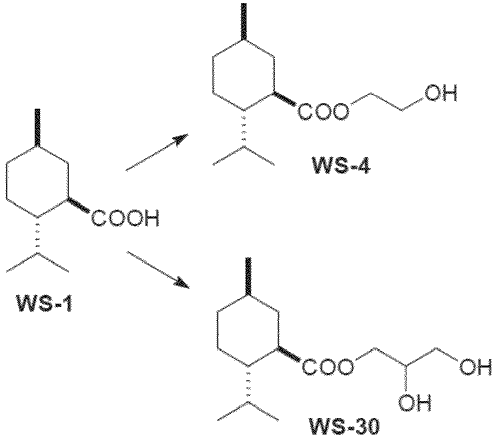

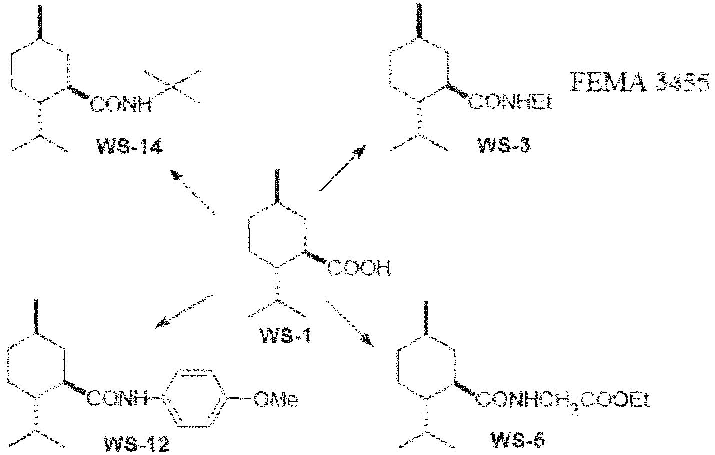

Die sich von WS-1 ableitenden Ester werden beispielsweise in US 4,157,384**,** die entsprechenden N-substituierten Amide in J. Soc. Cosmet. Chem. S. 185-200 (1978**)** beschrieben.

Nicht alle der oben beschriebenen Kühlstoffe sind bei Umgebungstemperatur fest und lassen sich pastillieren oder kompaktieren. Es ist aber sehr wohl möglich, Gemische der bei Umgebungstemperatur festen und flüssigen Kühlstoffe in das Verfahren einzusetzen und entsprechend feste Endprodukte zu erhalten.

Als Ausgangsstoff zur Durchführung des erfindungsgemäßen Verfahrens bevorzugt sind Schmelzen von racemischen Menthol, besonders bevorzugt von L-Menthol, wobei das geschmolzene Menthol natürlichen oder synthetischen Ursprungs sein kann und einen Enantiomerenüberschuß von üblicherweise mindestens 95, 96 oder 97 bis 100 %, bevorzugt 98, 98,5 oder 99 bis 99,9 % aufweist. Insbesondere eignen sich als Ausgangsstoffe im Rahmen des erfindungsgemäßen Verfahrens auch solche Schmelzen von L-Menthol, die einen Gehalt an L-Menthol von mindestens 95, 96 oder 97 Gew.-% oder darüber, bevorzugt mindestens 98 bis 100 Gew.-% und ganz besonders bevorzugt 98, 98,5 oder 99 bis 99,9 Gew.-% aufweisen (jeweils bezogen auf das Gesamtgewicht der Schmelze), neben Verunreinigungen wie beispielsweise Resten von Lösemitteln, Diastereomeren des L-Menthols oder Nebenprodukten aus Synthese- bzw. Isolierverfahren.

Dabei ist unter dem Begriff L-Menthol-Schmelze vorzugsweise solches L-Menthol zu verstehen, das weitgehend, d.h. zu mindestens 80 oder besser 85 Gew.-%, bevorzugt zu mindestens 90 oder 95 Gew.-% und ganz besonders bevorzugt zu mindestens 95, 96, 97, 98 oder 99 Gew.-% in geschmolzener Form vorliegt, wobei die restlichen Gewichtanteile die Menge an festem L-Menthol in der Schmelze ausmachen. Dabei kann es sich bei dem gegebenenfalls enthaltenen Anteil an festem Menthol in der Schmelze um solches handeln, das durch einen noch nicht vollständig abgeschlossenen Schmelzprozess des zur Bereitstellung der Schmelze eingesetzten Material noch in der Schmelze vorliegt oder dem vollständig bzw. teilweise geschmolzenen Menthol in fester Form zugesetzt wird, beispielsweise in Form von Kristallen von L-Menthol in der α-Modifikation. Solche auch als Impfkristalle zu bezeichnenden Kristalle von L- Menthol in der α-Modifikation können beispielsweise auf herkömmliche Weise durch Kristallisation von L-Menthol aus einer L-Menthol-haltigen Lösung oder Schmelze gewonnen werden.

Im Rahmen einer bevorzugten Ausführungsform setzt man solche Kristalle von L-Menthol in der α-Modifikation ein, die durch Behandlung der erfindungsgemäß einzusetzenden L-Menthol Schmelze in einem Kratzkühler gewonnen werden, wobei die Impfkristalle in situ in der zu verfestigenden L-Menthol Schmelze gebildet werden und ein zusätzlicher Arbeitsschritt vermieden wird.

### DURCHFÜHRUNG DES VERFAHRENS

Im Sinne des erfindungsgemäßen Verfahrens wird eine vorgekrätzte Schmelze einer Mentholverbindung mittels einer Pumpe beispielsweise einem so genannten Rotoform-System zugeführt.

Um eine möglichste vollständige Erstarrung der Schmelzen, vorzugsweise des L-Menthols in der α-Modifikation zu gewährleisten, wird die Schmelze vor dem Einbringen in den Rotoformer und der Aufgabe auf das Kühlband wie vorstehend beschrieben mit Impfkristallen versetzt. Dies kann beispielsweise durch Einrühren in ein Vorlagegefäß oder Aufstreuen von zuvor zerkleinerten Kristallen beispielsweise der α-Modifikation des L-Menthols auf eine eingesetzte L-Menthol-Schmelze (den flüssigen Kristallfilm) erreicht werden. Alternativ ist es möglich, das Kühlband mit α-Mentholkristallen zu bestreuen. In einer bevorzugten Ausführungsform der Erfindung wird die Impfung durch Passage der Schmelze durch einen unterhalb des Schmelzpunktes betriebenen Wärmetauscher erreicht, dessen Wände durch ein Abriebsorgan von aufkristallisiertem Material befreit werden. Dem Fachmann sind solche Anordnungen beispielsweise als so genannte "Kratzkühler" geläufig und werden beispielsweise in G. Arkenbout: "Melt Crystallization Technology", Technomic Publishing Co. 1995, p 230 beschrieben. Dementsprechend zeichnet sich eine bevorzugte Ausführungsform des erfindungsgemäßen Verfahrens dadurch aus, dass die Impfkristalle durch Behandlung der einzusetzenden Schmelzen in einem Kratzkühler gebildet werden.

Vorzugsweise wird eine vorgekrätzte Schmelze von Mentholverbindungen eingesetzt, die eine Temperatur im Bereich von etwa 40 bis etwa 60 °C und insbesondere etwa 43 bis 50 °C aufweist und/oder etwa 0,1 bis 12 Gew.-%, insbesondere etwa 1 bis etwa 5 Gew.-% Impfkristalle enthält. Besonders bevorzugt ist der Einsatz einer vorgekrätzten Schmelze, die etwa 0,1 bis 12 Gew.-% Impfkristalle von L-Menthol enthält. Es können auch bei Temperaturen von 42 bis 43 °C unterkühlte Mentholschmelzen eingesetzt werden. Um zu verhindern, dass sich im Kratzkühler Anbackungen bilden, die dann ungesteuert abplatzen und die Schmelztemperatur beeinflussen, empfiehlt es sich, den Kühler beispielsweise mit einer Begleitheizung auszustatten.

In einer bevorzugten Ausführungsform der Erfindung erfolgt die gleichmäßige Vertropfung mit Hilfe eines so genannten Rotoformers. Der Rotoformer besteht aus einem beheizten zylindrischen Innenkörper, der mit flüssigem Produkt beschickt wird, und einem mit zahlreichen Bohrungen versehenen Außenrohr, das sich konzentrisch um den Innenkörper dreht und dabei Produkttropfen auf die gesamte Länge eines Stahlbandkühlers ablegt. Ein im Innenkörper eingebautes System aus Umlenkungen und Düsen gewährleistet einen gleichmäßigen Druck über die gesamte Breite des Bauteils und somit einen gleichmäßigen Austritt des Produktes durch alle Bohrungen des Außenrohres. Dabei weisen alle Produkte, speziell die so erhältlichen Pastillen von einer Seite des Stahlbandes zur anderen eine gleichmäßige Größe auf. Die Umfangsgeschwindigkeit des Rotoformers ist vorzugsweise synchron zur Geschwindigkeit des Bandes: die Tropfen werden daher ohne Verformung abgelegt. Die während der Verfestigung und Kühlung freigesetzte Wärme wird durch das rostfreie Stahlband an das Kühlwasser, das gegen die Bandunterseite gesprüht wird, abgegeben. Das Wasser wird in Wannen gesammelt und der Rückkühlanlage zugeführt, in keinem Stadium kommt es mit dem Produkt in Berührung. Nach dem Ablegen des Tropfens auf dem Stahlband bleibt eine geringe Menge Produkt an den Außenrändern der Bohrungen des Außenrohres haften. Ein beheizbarer Einweiser drückt dieses Produkt in einen inneren Spalt im Rotoformer, von wo es mit dem Originalprodukt vermischt und erneut auf das Stahlband aufgegeben wird. Um eine Verstopfung des Rotoformers zu vermeiden, empfiehlt sich hier beispielsweise der Einsatz einer Wärmestauhaube. Entsprechende Kombinationen aus Rotoformer und Stahlbandkühler werden beispielsweise von der Firma Sandvik Process Systems GmbH, D-70736 Fellbach im Handel angeboten. Eine sehr ähnliche Technologie wird beispielsweise unter der Bezeichnung Rollomat von der Firma Kaiser Process & Belt Technology GmbH, D-47800 Krefeld angeboten. Grundsätzlich geeignet sind auch rotierende und vibrierende Lochplatten, sofern die Viskosität (entsprechend dem Feststoffanteil in der Schmelze) der Schmelztröpfchen nicht zu hoch ist.

Vorzugsweise werden die Schmelztropfen vom Rotoformer auf ein gekühltes Band, insbesondere ein gekühltes Stahlband abgelegt welches mehrere Kühlzonen aufweisen kann, die unabhängig voneinander temperiert werden können, beispielsweise auf Temperaturen unterhalb des Schmelz- oder Erstarrungspunktes, für L-Menthol im Bereich von etwa 5 bis etwa 42 °C. Typisch sind beispielsweise Kühlbänder mit drei Kühlzonen, von denen die ersten beiden Temperaturen von etwa 25 bis 30 und die letzte von etwa 15 bis 20 °C aufweisen. Beispielsweise kommen Kühlbänder zum Einsatz, die eine Länge von etwa 2 bis etwa 20 m und eine Breite von etwa 10 bis etwa 200 cm aufweisen. Die Laufgeschwindigkeit der Kühlbänder wird dann vorteilhafterweise so eingestellt, dass unter Berücksichtigung der oben genannten Geometrie der Bänder auf diese Weise eine Kühlzeit eingehalten wird, die eine vollständige Kristallisation der Schmelzen sicherstellt. In Abhängigkeit der gewünschten Kapazität können selbstverständlich auch größere Einheiten eingesetzt werden, wobei sich die Kapazität proportional zur Breite des Kühlbandes steht und sich die Verweilzeit aus Länge und Geschwindigkeit des Kühlbandes ergibt. Grundsätzlich kann das Verfahren auch auf Anlagen durchgeführt werden, die eine Kapazität von 50 bis 1.000 kg/h oder größer aufweisen.

Anschließend werden die festen Kühlstoffe vom Band abgenommen, was beispielsweise mit Hilfe eines Messers erfolgt. Die Abnahme kann dabei entweder nach der einfachen Kühllänge oder unter Zuhilfenahme nach weiterem Verbleib auf dem Kühlband nahe der Aufgabestelle erfolgen. Das Material kann zum Reifen auch länger auf dem Band verbleiben und in einem untertemperierten oder nachtemperierten Bereich verbleiben.

Anstelle eines Kühlbandes können auch Kühlteller oder dergleichen eingesetzt werden, wie sie beispielsweise als Pastillierhilfen von der Firma Andritz Gouda, NL-2740 Waddingxveen vertrieben werden.

Aufgabe des Verfahrens ist es möglichst durchkristallisierte feste Kühlstoffe zur Verfügung zu stellen, die während der Lagerung mechanisch stabil bleiben. Dabei gilt es zu berücksichtigen, dass auch die thermodynamisch stabilste Form des L-Menthols sublimiert. Da Sublimation ein Vorgang an der Oberfläche der Partikel ist, ist es vorteilhaft, wenn die Partikel ein kleines Oberflächen-zu-Volumenverhältnis aufweisen. Denn durch Sublimation bilden sich bei Lagerung Brückenverbindungen zwischen den Partikeln, was das Verbacken erklärt. Zudem sind Partikel mit teilweise gewölbten Oberflächen zu bevorzugen, bei denen in der Schüttung weniger Kontaktpunkte auftreten.

Im Rahmen der vorliegenden Erfindung - soweit sich diese auf L-Menthol als Einsatzstoff bezieht - wird die Erstarrung bzw. Kristallisation der eingesetzten L-Menthol-Schmelze vorzugsweise erst dann als abgeschlossen betrachtet, wenn das erhaltene L-Menthol in fester Form zu mindestens etwa 80 Gew.-% oder besser 85 bis 100 Gew.-%, bevorzugt zu 90 bis 100 Gew.-%, bevorzugt zu 95 oder 97 bis 99,5 Gew.-% und ganz besonders bevorzugt zu 98 bis 99 Gew.-% in der α-Modifikation vorliegt. Derartiges L-Menthol weist nur noch in geringem Maß Veränderungen durch Umwandlung des Materials in die thermodynamisch stabilste Modifikation auf und verändert sich daher bezüglich seiner Oberflächenbeschaffenheit, wenn überhaupt, nur noch in geringem Umfang.

Die erfindungsgemäßen Partikel zeichnen sich dadurch aus, dass sie nur noch geringe Feinanteile enthalten, ein günstiges Oberflächen/Volumenverhältnis aufweisen und gleichzeitig gewölbte aber glatte Oberflächen besitzen, die in der Schüttung möglichst kleine Kontaktflächen ergeben und unempfindlich gegen Abrieb sind und zudem möglichst wenig oder keine Bruchkannten aufweisen.

Ein weiterer bevorzugter Gegenstand der Erfindung betrifft daher Mentholpartikel in Pastillenform mit einer gewölbten und einer flachen Seite, die einen Durchmesser von etwa 1 bis etwa 20 mm, bevorzugt 5 bis 12 mm aufweisen und sich des Weiteren dadurch auszeichnen, dass sie
(i) einen Feinanteil (d.h. einen Anteil von Partikeln mit einem mittleren Durchmesser kleiner 1,6 mm) von weniger als 5 Gew.-%, vorzugsweise weniger als 2 Gew.-% und insbesondere weniger als 1 Gew.-%, ganz bevorzugt weniger als 0,5 Gew.-%, besonders bevorzugt weniger als 0,1 Gew.-% aufweisen, und/oder
(ii) einen alpha-Mentholgehalt von mindestens 80 Gew.-%, vorzugsweise etwa 85 bis etwa 99 Gew.-% und insbesondere etwa 90 bis etwa 95 Gew.-% besitzen; und/oder
(iii) ein Oberflächen-zu-Volumenverhältnis von kleiner als 2 1/mm, vorzugsweise kleiner als 1,5 1/mm und besonders bevorzugt kleiner als 1,0 1/mm aufweisen.
(iv) gewölbte Oberflächen aufweisen, so dass das Verhältnis von planer Oberfläche zur Gesamtoberfläche des Partikels maximal 60 %, vorzugsweise weniger als 50 % und bevorzugt weniger als 40 % beträgt.

Die jeweils vorliegende Modifikation des erhaltenen verfestigten L-Menthols und somit der Abschluss des Erstarrungsvorgangs kann mit dem Fachmann bekannten Verfahren wie Röntgenbeugung oder Pulverdiffraktometrie ermittelt werden (s. z.B. Joel Bernstein, "Polymorphism in Molecular Crystals", Oxford University Press 2002, S. 94-150).

### GEWERBLICHE ANWENDBARKEIT

Ein weiterer Gegenstand der Erfindung betrifft Kühlstoffe in fester Form, die nach dem oben geschilderten Verfahren erhältlich sind und in Form von Schuppen oder vorzugsweise Pastillen vorliegen. Mit Hilfe des erfindungsgemäßen Verfahrens ist beispielsweise die Herstellung von Pastillen mit Durchmessern von etwa 1 bis etwa 20 mm, vorzugsweise etwa 5 bis etwa 12 mm problemlos möglich. Da die Partikel nach Lagerung auch leicht in Lösung gebracht werden sollen, stellt die angegebene Partikelgröße eine optimale Balance zwischen Löslichkeit einerseits und Verbackungsneigung andererseits dar.

Auch diese Kühlstoffen ist vorzugsweise gemeinsam, dass sie
(i) einen Feinanteil (d.h. einen Anteil von Partikeln mit einem mittleren Durchmesser kleiner 1,6 mm) von weniger als 5 Gew.-%, vorzugsweise weniger als 2 Gew.-% und insbesondere weniger als 1 Gew.-%, ganz bevorzugt weniger als 0,5 Gew.-%, besonders bevorzugt weniger als 0,1 Gew.-% aufweisen, und/oder
(ii) einen alpha-Mentholgehalt von mindestens 80 Gew.-%, vorzugsweise etwa 85 bis etwa 99 Gew.-% und insbesondere etwa 90 bis etwa 95 Gew.-% besitzen; und/oder
(iii) ein Oberflächen-zu-Volumenverhältnis von kleiner als 2 1/mm, vorzugsweise kleiner als 1,5 1/mm und besonders bevorzugt kleiner als 1,0 1/mm aufweisen.
(iv) gewölbte Oberflächen aufweisen, so dass das Verhältnis von planer Oberfläche zur Gesamtoberfläche des Partikels maximal 60 %, vorzugsweise weniger als 50 % und bevorzugt weniger als 40 % beträgt.

Ein letzter Gegenstand der vorliegenden Erfindung ist die Verwendung der festen Kühlstoffe bzw. der Mentholpartikel in kosmetischen oder pharmazeutischen Zubereitungen sowie Nahrungsmitteln.

### BEISPIELE

### HERSTELLBEISPIELE

In den folgenden Ausführungsbeispielen sollte die Herstellung von Pastillen mit einem optimalen Oberflächen-zu-Volumenverhältnis und guten Verbackungseigenschaften hergestellt und untersucht werden. Ebenfalls sollten Verfahrensbedingungen ermittelt werden, die die Herstellung von vollständig durchkristallisiertem Material sicherstellt. Eine Nachkristallisation im abgepackten Zustand sollte verhindert werden.

### VERSUCHSAUFBAU UND VERFAHRENSDURCHFÜHRUNG

Die Versuche wurden auf einem Stahlbandkühler mit Rotoformer und vorgeschaltetem Kratzkühler durchgeführt wie in **Abbildung A** schematisch dargestellt. Dabei bedeuten die Bezugszeichen Folgendes:

| | | | |
|---|---|---|---|
| 1 | Eduktbehälter | 6 | Eduktrückführung |
| 2 | Eduktpumpe | 7 | Rotoformer |
| 3 | Extruder | 8 | Kühlband mit drei Kühlzonen T1, T2, T3 |
| 4 | Wärmetauscher | 9 | Granulator |
| 5 | Ventil | 10 | Produktvorlage |

Eine im Kratzkühler vorgekrätzte Schmelze (d.h. eine Suspension von Impfkristallen in Menthol) wurde hierbei über einen Rotoformer auf ein vorgekühltes Stahlband aufgegeben. Die Kühlbandlänge betrug 12,5 m, die Bandbreite 600 mm. Das Kühlband hatte drei Kühlzonen, welche unabhängig voneinander temperiert werden konnten. Die Abnahme vom Kühlband erfolgte durch ein Messer, entweder nach der einfachen Kühllänge oder unter Zuhilfenahme des Bandrücklaufs nahe der Aufgabestelle. Hierdurch erfuhr das Material auf den 12,5 m des Bandrücklaufs eine zusätzliche Abkühlung. Die Laufgeschwindigkeit des Kühlbandes (und somit die Kapazität des Kühlbandes) wurde über die Versuche nur unwesentlich geändert, wodurch über die Versuche ein Durchsatz im Bereich von 150 -165 kg/h resultierte. Das erhaltene Material wurde mit einer Rüttelsiebmaschine (Fa. Allgaier; Siebweite: 1,6 mm und 1,25 mm) von anhaftenden Feinanteilen getrennt. Die Abladetemperatur des Materials wurde durch Messung in einem Dewargefäß mit Thermoelement bestimmt. Die Änderung der Temperatur nach Abnahme vom Band wird im Folgenden als Nachkristallisationswärme bezeichnet. Der Durchsatz wurde mit Stoppuhr und Waage im Mittelteil eines Versuchsdurchlaufes bestimmt. Pro Versuch wurden etwa 20 bis 30 kg Pastillen als Vorlauf genommen. Die erhaltenen Materialien wurden während des Versuchs in F1 Kartons mit PE-Innenbeutel (Symrise Standard Packmittel für Menthol kompaktiert) verpackt.

### BEISPIEL 1

Als Starttemperatur wurden 30°C auf T1 und T2 gewählt, da diese Temperatur des Metallbandes nahe an der Erstarrungstemperatur der α-Modifikation liegt (siehe Bild 1) und bei einer überlagerten spontanen Kristallisation nur wenig γ-Modifikation zu erwarten war. Die Erstarrungstemperatur in der Pastille sollte durch den schlechteren Wärmeübergang durch das erstarrte Menthol höher liegen und somit bevorzugt zur Bildung von α-Modifikation führen. Auf T3 wurden 15°C gewählt um den Wärmetransport durch das bereits verfestigte Menthol zu forcieren und so eine vollständige Kristallisation zu gewährleisten. Die Versuchsbedingungen sind in **Tabelle 1** wiedergegeben

**Tabelle 1**

| Versuchsbedingungen | | |
|---|---|---|
| **Temperaturzonen des Kühlband** | T1: 30 °C; T2: 30 °C; T3: 15 °C | |
| **Temperatur Kratzkühler** | 41,5 °C | |
| **Durchsatz** | 150 kg/h | |
| **Gewichte (Kartons)** | 15,6 kg / 12,45 kg / 19,50 kg / 21,05 kg / 20,6 kg | |
| **Feinanteile** | 41,8 g auf 89,2 kg (469 g / t Pastillen) | |
| **Abladetemperatur** | 25,2 °C | |
| **Nachkristallisationswärme** | 1°C über 1 h | Raumtemperatur: 27°C |

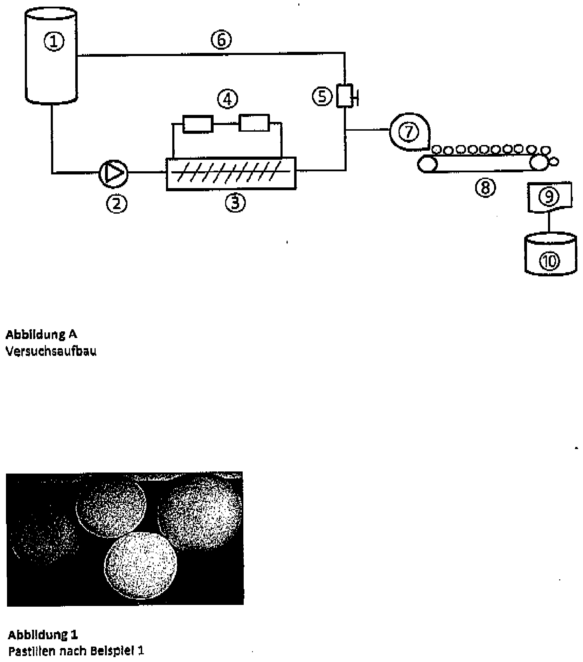

Nach kurzem Vorlauf wurden rein weiße Pastillen erhalten **(****Abbildung 1****)** Die Pastillen waren vollständig durchkristallisiert und nur schwer mit Spatel/Messer zu zerteilen.

### BEISPIEL 2

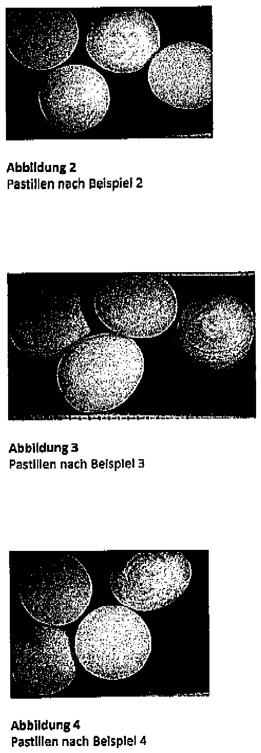

Nach Durchlaufen des Kühlbandes waren die Pastillen bei T2 noch nicht vollständig durchkristallisiert. Am Ende des Kühlbandes bei T3 waren die Pastillen am oberen Ende noch leicht weich und mit dem Messer gut zu zerteilen. Die Versuchsbedingungen sind in **Tabelle 2** wiedergegeben; die Pastillen sind in **Abbildung 2** zu sehen.

**Tabelle 2**

| Versuchsbedingungen | | |
|---|---|---|
| **Temperaturzonen des Kühlband** | T1: 30 °C; T2: 30 °C; T3: 18 °C | |
| **Temperatur Kratzkühler** | 41,4 - 41,6 °C | |
| **Durchsatz** | 165 kg/h | |
| **Gewichte (Kartons)** | 19,82 kg / 20,48 kg / 20,93 kg / 20,27 kg | |
| **Feinanteile** | 106,4 g auf 81,5 kg (1305 g / t Pastillen) | |
| **Abladetemperatur** | 24,4 °C | |
| **Nachkristallisationswärme** | 1°C über 30 min | Raumtemperatur: 27°C |

### BEISPIEL 3

Im Laufe von Versuch 3 wurde das Abnahmemesser am Ende des Kühlbandes entfernt. Der Bandrücklauf wurde als zusätzliche Nachkühlstrecke verwendet. Die Pastillen waren nach Abnahme hart und durchkristallisiert. Die Versuchsbedingungen sind in **Tabelle 3** wiedergegeben; die Pastillen sind in **Abbildung 3** zu sehen.

**Tabelle 3**

| Versuchsbedingungen | | |
|---|---|---|
| **Temperaturzonen des Kühlband** | T1: 30 °C; T2: 30 °C; T3: 18 °C; Bandrücklauf Nachkühlung | |
| **Temperatur Kratzkühler** | 41,4 - 41,6 °C | |
| **Durchsatz** | 165 kg/h | |
| **Gewichte (Kartons)** | 20,57 kg / 20,49 kg / 19,36 kg | |
| **Feinanteile** | 48,6 g auf 60,42 kg (804 g / t Pastillen) | |
| **Abladetemperatur** | 24,4 °C | |
| **Nachkristallisationswärme** | 1°C über 30 min | Raumtemperatur: 27°C |

### BEISPIEL 4

Die Pastillen waren vergleichbar zu Beispiel 1. Die Versuchsbedingungen sind in **Tabelle 4** wiedergegeben; die Pastillen sind in **Abbildung 4** zu sehen.

**Tabelle 4**

| Versuchsbedingungen | | |
|---|---|---|
| **Temperaturzonen des Kühlband** | T1: 30 °C; T2: 30 °C; T3: 15 °C | |
| **Temperatur Kratzkühler** | 41,5 - 41,6 °C | |
| **Durchsatz** | 150 kg/h | |
| **Gewichte (Kartons)** | 18,85 kg / 18,75 kg / 19,45 kg / 19,65 kg | |
| **Feinanteile** | 106,1 g auf 76,7 kg (1383 g / t Pastillen) | |
| **Abladetemperatur** | 25,2 °C | |
| **Nachkristallisationswärme** | 1,0°C über 30 min | Raumtemperatur: 28°C |

### BEISPIEL 5

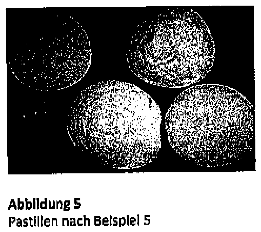

Im Laufe von Beispiel 5 wurde das Abnahmemesser am Ende des Kühlbandes entfernt. Der Bandrücklauf wurde als zusätzliche Nachkühlstrecke verwendet. Die Pastillen waren nach Abnahme hart und durchkristallisiert. Die Versuchsbedingungen sind in **Tabelle 5** wiedergegeben; die Pastillen sind in **Abbildung 5** zu sehen.

**Tabelle 5**

| Versuchsbedingungen | | |
|---|---|---|
| **Temperaturzonen des Kühlband** | T1: 30 °C; T2: 30 °C; T3: 15 °C; Bandrücklauf Nachkühlung | |
| **Temperatur Kratzkühler** | 41,4 - 41,6 °C | |
| **Durchsatz** | 165 kg/h | |
| **Gewichte (Kartons)** | 15,4 kg | |
| **Abladetemperatur** | 22,2 °C | |
| **Nachkristallisationswärme** | 1,0°C über 30 min | Raumtemperatur: 28°C |

### BEISPIEL 6, VERGLEICHSBEISPIEL V1

Beispiel 1 wurde wiederholt, die Kühlung jedoch mit Hilfe eines Doppelbandkühlers mit gekühlten Stahloberflächen und einer Spaltweite von 0,3 cm (Länge 12 m, Breite 35 cm) durchgeführt. Der Doppelbandkühler wies ebenfalls 3 Kühlzonen (30°C, 30 °C, 15 °C) auf; das Produkt wurde in Form von Schuppen mit Hilfe eines Messers abgenommen.

Jeweils 5 kg der Pastillen nach dem erfindungsgemäßen Beispiel 1 (Durchmesser: 5 mm) sowie dem Vergleichsbeispiel V1 wurden in Kunststoffbeutel abgefüllt und diese in Kartons über einen Zeitraum von 6 Wochen bei 20 °C gelagert. Die Ergebnisse sind in **Tabelle 6** zusammengefasst.

**Tabelle 6**

| Lagertests | | |
|---|---|---|
| **Lagerzeit** | **Beispiel 1** | **Vergleichsbeispiel V1** |
| 1 Woche | Material an der Oberfläche leicht vereinzelbar. Keine Verklumpung. | Material an der Oberfläche leicht vereinzelbar. Keine Verklumpung. |
| 2 Wochen | Material an der Oberfläche leicht vereinzelbar. In der Mitte der Füllung geringe Menge zu Klumpen verbacken, die sich von Hand vereinzeln lassen. | Material an der Oberfläche vereinzelbar. In der Mitte der Füllung Klumpenbildung, die sich mit einer Schaufel vereinzeln lassen. |
| 3 Wochen | Material an der Oberfläche leicht vereinzelbar. In der Mitte der Füllung geringe Menge zu Klumpen verbacken, die sich von Hand vereinzeln lassen. | Material an der Oberfläche vereinzelbar. In der Mitte der Füllung deutliche Klumpenbildung, die sich mit einer Schaufel vereinzeln lassen. |
| 4 Wochen | Material an der Oberfläche leicht vereinzelbar. In der Mitte der Füllung geringe Menge zu Klumpen verbacken, die sich mit von Hand vereinzeln lassen. | Material an der Oberfläche vereinzelbar. In der Mitte der Füllung deutliche Klumpenbildung, die sich mit einer Schaufel vereinzeln lassen. |
| 5 Wochen | Material an der Oberfläche vereinzelbar. In der Mitte der Füllung Klumpenbildung, die sich mit einer Schaufel vereinzeln lassen. | Material an der Oberfläche schwer vereinzelbar. In der Mitte der Füllung deutliche Klumpenbildung, die sich mit einer Schaufel vereinzeln lassen. An der Oberfläche geringe Nadelbildung durch Sublimation. |
| 6 Wochen | Material an der Oberfläche vereinzelbar. In der Mitte der Füllung deutliche Klumpenbildung, die sich mit einer Schaufel vereinzeln lassen. An der Oberfläche geringe Nadelbildung durch Sublimation. | 80 % verklumpt, deutliche Nadelbildung durch Sublimation. |

### BEISPIEL 7

Um zu belegen, dass die Kristallmodifikation alleine nicht ausschlaggebend für die Verbackungsneigung ist, wurden 20 kg über 8 Monate abgelagertes L-Menthol, das vollständig in seiner α-Modifikation vorlag, über eine Siebmühle mit 3 mm Sieblocheinsatz zu Kristallpulver zerkleinert. Dabei wurde keine Temperaturerhöhung durch den Zerkleinerungsvorgang gemessen. Anschließend wurde das Kristallpulver wieder gelagert. Nach bereits zwei Wochen war das Pulver zu einem Block verklumpt, der nur durch starkes Kneten lokal aufgelockert werden konnte.

## Patentansprüche

1. Verfahren zur Herstellung von festen Kühlstoffen, umfassend die folgenden Schritte:
(a) Versetzen einer Schmelze von Mentholverbindungen mit Impfkristallen, und
(b) gleichmäßige Vertropfung der so vorgekrätzten Schmelze auf eine vorgekühlte Fläche.

2. Verfahren nach Anspruch 1, **dadurch gekennzeichnet, dass** die Mentholverbindungen ausgewählt sind aus der Gruppe, die gebildet wird von Menthol, Racemischem Menthol, Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

3. Verfahren nach Anspruch 2, **dadurch gekennzeichnet, dass** man eine vorgekrätzte Schmelze von Mentholverbindungen einsetzt, die eine Temperatur im Bereich von 40 bis 60 °C aufweist.

4. Verfahren nach mindestens einem der Ansprüche 1 bis 3, **dadurch gekennzeichnet, dass** man eine vorgekrätzte Schmelze einsetzt, die 0,1 bis 12 Gew.-% Impfkristalle enthält.

5. Verfahren nach Anspruch 4, **dadurch gekennzeichnet, dass** man eine vorgekrätzte Schmelze einsetzt, die etwa 0,1 bis 12 Gew.-% Impfkristalle von L-Menthol enthält.

6. Verfahren nach mindestens einem der Ansprüche 1 bis 5, **dadurch gekennzeichnet, dass** man die Schmelze auf einem Stahlband abkühlt, welches mindestens zwei Kühlzonen aufweist, die unabhängig voneinander temperiert werden können.

7. Verfahren nach mindestens einem der Ansprüche 1 bis 6, **dadurch gekennzeichnet, dass** man die Schmelze auf einem Stahlband abkühlt, wobei die Kühlzonen jeweils unabhängig voneinander Temperaturen unterhalb des jeweiligen Schmelzpunktes, für L-Menthol im Bereich von etwa 15 bis etwa 42 °C aufweisen.

8. Mentholpartikel erhältlich nach dem Verfahren nach mindestens einem der Ansprüche 1 bis 7, **dadurch gekennzeichnet, dass** sie
(i) einen Feinanteil von weniger als 5 Gew.-% aufweisen,
(ii) einen alpha-Mentholgehalt von mindestens 80 Gew.-%; und
(iii) ein Oberflächen-zu-Volumenverhältnis von kleiner als 2 1/mm besitzen sowie
(iv) gewölbte Oberflächen aufweisen, so dass das Verhältnis von planer Oberfläche zur Gesamtoberfläche des Partikels maximal 60 % beträgt.

9. Partikel nach Anspruch 8, **dadurch gekennzeichnet, dass** sie einen Feinanteil von weniger als 2 Gew.-% aufweisen.

10. Partikel nach den Ansprüchen 8 und/oder 9, **dadurch gekennzeichnet, dass** sie einen α-Mentholgehalt von etwa 85 bis 99 Gew.-% besitzen.

11. Partikel nach mindestens einem der Ansprüche 8 bis 10, **dadurch gekennzeichnet, dass** ein Oberflächen-zu-Volumenverhältnis von kleiner 1,5 1/mm aufweisen.

12. Verwendung von Mentholpartikeln nach den Ansprüchen 8 bis 11 in kosmetischen oder pharmazeutischen Zubereitungen oder Nahrungsmitteln.

## Claims

1. A process for the production of solid refrigerants comprising the following steps:
(a) adding seed crystals to a melt of menthol compounds, and
(b) consistent dripping of the thus pre-scraped melt onto a pre-cooled surface.

2. The process according to claim 1, **characterized in that** the menthol compounds are selected from the group formed by menthol, racemic menthol, menthol methyl ether, menthone glyceryl acetal (FEMA GRAS 3807), menthone glyceryl ketal (FEMA GRAS 3808), menthyl lactate (FEMA GRAS 3748), menthol ethylene glycol carbonate (FEMA GRAS 3805), Menthol Propylene Glycol Carbonate (FEMA GRAS 3806), Menthyl N-ethyloxamate, Monomethyl Succinate (FEMA GRAS 3810), Monomenthyl Glutamate (FEMA GRAS 4006), Menthoxy-1, 2-propanediol (FEMA GRAS 3784), menthoxy-2-methyl-1,2-propanediol (FEMA GRAS 3849) as well as the menthanocarboxylic acid esters and amides WS-3, WS-4, WS-5, WS-12, WS-14 and WS-30 and their mixtures.

3. The process according to claim 2, **characterized in that** a pre-scratched melt of menthol compounds is used, which has a temperature in the range of 40 to 60 °C.

4. The process according to at least one of claims 1 to 3, **characterized in that** a precreated melt containing 0.1 to 12% by weight of seed crystals is used.

5. The process according to claim 4, **characterized in that** a pre-scratched melt is used which contains about 0.1 to 12% by weight of seed crystals of L-menthol.

6. The process according to at least one of claims 1 to 5, **characterized in that** the melt is cooled on a steel strip which has at least two cooling zones which can be tempered independently of one another.

7. The process according to at least one of Claims 1 to 6, which comprises cooling the melt on a steel strip, the cooling zones each having, independently of one another, temperatures below the respective melting point, for L-menthol in the range from about 15 to about 42°C.

8. Menthol particles obtainable by the process according to at least one of Claims 1 to 7, **characterized in that** they
(i) have a fines content of less than 5 % by weight
(ii) an alpha-menthol content of at least 80% by weight; and
(iii) have a surface to volume ratio of less than 2 1/mm; and
(iv) have curved surfaces so that the ratio of the flat surface to the total surface of the particle is at most 60%.

9. The particles according to claim 8, **characterized in that** they have a fine fraction of less than 2% by weight.

10. The particles according to claims 8 and/or 9, **characterized in that** they have a α-menthol content of approximately 85 to 99% by weight.

11. The particles according to at least one of claims 8 to 10, **characterized in that** they have a surface-to-volume ratio of less than 1.5 1/mm.

12. Use of menthol particles according to claims 8 to 11 in cosmetic or pharmaceutical preparations or foodstuffs.

## Revendications

1. Un procédé de production de réfrigérants solides comprenant les étapes suivantes :
(a) l'ajout de cristaux d'ensemencement à une fusion de composés de menthol, et
(b) l'égouttage uniforme de la masse fondue ainsi raclée sur une surface prérefroidie.

2. Procédé selon la revendication 1, **caractérisé en ce que** les composés de menthol sont choisis dans le groupe formé par le menthol, le menthol racémique, l'éther méthylique de menthol, le menthone glycéryl acétal (FEMA GRAS 3807), le menthone glyceryl cétal (FEMA GRAS 3808), le lactate de menthyle (FEMA GRAS 3748), le menthol éthylène glycol carbonate (FEMA GRAS 3805), Carbonate de menthol propylène glycol (FEMA GRAS 3806), N-éthyl-oxamate de menthyle, Succinate monométhylique (FEMA GRAS 3810), Glutamate de mono-menthyle (FEMA GRAS 4006), Menthoxy-1, 2-propanediol (FEMA GRAS 3784), le menthoxy-2-méthyl-1,2-propanediol (FEMA GRAS 3849) ainsi que les esters et amides d'acide menthanocarboxylique WS-3, WS-4, WS-5, WS-12, WS-14 et WS-30 et leurs mélanges.

3. Procédé selon la revendication 2, **caractérisé en ce qu'**on utilise une masse fondue pré-gratté de composés de menthol qui a une température dans la gamme de 40 à 60°C.

4. Procédé selon au moins une des revendications 1 à 3, **caractérisé en ce que** l'on utilise une masse fondue pré-creusée contenant 0,1 à 12 % en poids de cristaux d'ensemencement.

5. Procédé selon la revendication 4, **caractérisé en ce que** l'on utilise une masse fondue pré-gratté qui contient environ 0,1 à 12 % en poids de cristaux d'ensemencement de L-menthol.

6. Procédé selon au moins une des revendications 1 à 5, **caractérisé en ce que** la masse fondue est refroidie sur une bande d'acier qui présente au moins deux zones de refroidissement pouvant être trempées indépendamment l'une de l'autre.

7. Procédé selon au moins une des revendications 1 à 6, qui consiste à refroidir la masse fondue sur une bande d'acier, les zones de refroidissement ayant chacune, indépendamment les unes des autres, des températures inférieures au point de fusion respectif, pour le L-menthol, dans la plage d'environ 15 à environ 42°C.

8. Particules de menthol pouvant être obtenues par le procédé selon au moins une des revendications 1 à 7, **caractérisées en ce qu'**elles
(i) ont une teneur en amendes inférieure à 5 % en poids
(ii) une teneur en alpha-menthol d'au moins 80 % en poids ; et
(iii) avoir un rapport surface/volume inférieur à 2 1/mm ; et
(iv) avoir des surfaces convexes telles que le rapport entre la surface plane et la surface totale de la particule ne dépasse pas 60 %.

9. Les particules selon la revendication 8, **caractérisées en ce qu'**elles présentent une fraction fine inférieure à 2 % en poids.

10. Les particules selon les revendications 8 et/ou 9, **caractérisées en ce qu'**elles ont une teneur en α-menthol d'environ 85 à 99 % en poids.

11. Les particules selon au moins une des revendications 8 à 10, **caractérisées en ce qu'**elles ont un rapport surface/volume inférieur à 1,5 1/mm.

12. L'utilisation de particules de menthol selon les revendications 8 à 11 dans des préparations cosmétiques ou pharmaceutiques ou des denrées alimentaires.
